# EUROPÄISCHE PATENTANMELDUNG

(11) **EP 0 824 904 A2**
(43) Veröffentlichungstag der Anmeldung: **25.02.1998**
(21) Anmeldenummer: 97112565.3
(22) Anmeldetag: 22.07.1997
(51) Int. Cl.: A61F 2/46

(54) **Anordnung zum Einsetzen einer femoralen Knie-Gleitflächen-Endoprothese**

(30) Priorität: 19.08.1996 DE 29614349 U
(71) Anmelder: Waldemar Link (GmbH & Co.), D-22339 Hamburg (DE)
(72) Erfinder: Keller, Arnold, 23863 Kayhude (DE)
(74) Vertreter: Glawe, Delfs, Moll & Partner

(57) **Zusammenfassung**

Anordnung zum Einsetzen einer femoralen Knie-Gleitflächen-Endoprothese bestehend aus einem Einsetzgerät (6) sowie zusammenwirkenden Halteeinrichtungen an diesem und der Prothese (1, 2). Das Einsetzgerät (6) bildet zwei Aufnahmen (8, 9) für unverbundene Condylenprothesenteile (1, 2). Jede Aufnahme (8, 9) wirkt mit dem zugehörigen Condylenprothesenteil (1, 2) über mindestens ein Paar von Führungsschienen (5, 10) zusammen, von denen eine Führungsschiene (5) am Seitenrand des Condylenprothesenteils (1 bzw. 2) angeordnet ist. Diese Führungsschienen laufen zweckmäßigerweise etwa in AP-Richtung und sind zweckmäßigerweise beidseitig an den Aufnahmen (8, 9) und Prothesenteilen (1, 2) vorgesehen. Eine Arretierungseinrichtung (11) kann zum Arretieren der Prothesenteile (1, 2) in den Aufnahmen (8, 9) vorgesehen sein.

## Beschreibung

Zum Ersatz der femoralen (oberschenkelseitigen) Condylengleitflächen des Kniegelenks sind Gleitflächenprothesen bekannt, die verhältnismäßig dünn ausgeführt sind und daher nur geringe Resektion des Knorpel- und Knochenmaterials verlangen und in manchen Fällen die teilweise oder vollständige Erhaltung der harten Knochenrinde gestatten. In der Regel werden die den beiden Condylen zugeordneten Prothesengleitflächen durch einen schildartigen Teil verbunden, der auf der Vorderseite hochragt und eine Gleitfläche für die Kniescheibe bildet. Eine solche Verbindung der Condylengleitflächen sichert ihre korrekt aufeinander abgestimmte Position. In vielen Fällen ist der Ersatz der Kniescheibengleitfläche nicht erforderlich. Man könnte sich dann mit einer einfacheren Verbindung der beiden Condylengleitflächen begnügen. Jedoch hat die Erfahrung gezeigt, daß eine einfachere Verbindung den Gebrauchsbeanspruchungen oftmals nicht gewachsen ist und bricht. Man kann statt dessen zu unverbundenen Condylengleitflächen greifen, die aber den Nachteil haben, daß ihre korrekte gegenseitige Positionierung freihändig kaum möglich ist.

Zum Einsetzen von gegenseitig positionierten Tibiaplateaus ist ein Werkzeug bekannt (Prospekt "Schalen-Kniegelenkprothesen-System SKI" der Firma Interplanta GmbH, Hamburg), das zwei zueinander parallele, je in eine Bohrung eines der Plateaus einschiebbare Stifte und zwei quer dazu verlaufende, jeweils in eine Querbohrung fassende Dornen aufweist. Eine solche Lösung ist bei Condylen-Gleitflächenprothesen nicht anwendbar, vor allem, weil die Lage der Gleitfläche die Unterbringung der genannten Bohrungen nicht gestattet.

Der Erfindung liegt daher die Aufgabe zugrunde, eine Anordnung zu schaffen, die das Einsetzen einer femoralen Knie-Gleitflächen-Endoprothese mit korrekt aufeinander abgestimmten Condylengleitflächenteilen ermöglicht.

Die erfindungsgemäße Lösung besteht in den Merkmalen des Anspruchs 1 und vorzugsweise auch denjenigen der Unteransprüche.

Die Erfindung setzt voraus, daß unverbundene Gleitflächenprothesenteile verwendet werden. Deren korrekte gegenseitige Positionierung wird durch entsprechend ausgerichtetete Halterungseinrichtungen in einem Einsetzinstrument gewährleistet, das zwei Aufnahmen für die Prothesenteile bildet. Diese sind mit zu den Halterungsteilen des Einsetzgeräts passenden Halterungselementen ausgerüstet. Diese bestehen aus jeweils einem Schienenpaar, das vorzugsweise etwa in AP-Richtung (Anterior-Posterior-Richtung) angeordnet ist. Das ist die Richtung, die bei gestrecktem Knie von vorne nach hinten verläuft, also in einer Sagittalebene lotrecht zur Richtung des Oberschenkelknochens. Dies Merkmal gestattet es, das Werkzeug mit den jeweils U-förmigen Aufnahmen nach dem Einsetzen der Prothesenteile nach vorne wegzuziehen.

Die Schienenpaare sind zweckmäßigerweise beidseitig an den Aufnahmen und den Prothesenteilen vorgesehen. Statische Eindeutigkeit ist aber auch dann schon gegeben, wenn lediglich an einer Seite der Aufnahmen und der Prothesenteile ein Schienenpaar angeordnet ist, während auf der anderen Seite eine punktförmige oder sonstwie geartete Halterung vorgesehen ist. Beispielsweise kann am Rand des Prothesenteils eine Bohrung oder Körnereinsenkung vorgesehen sein, in die die Spitze einer an der Aufnahme vorgesehenen Schraube eingreift, die zum Lösen des Geräts von den Prothesenteilen gelöst wird.

In jedem Fall, also auch dann, wenn die Schienenanordnungen beidseitig vorgesehen sind, sollte eine Arretierungseinrichtung vorgesehen sein, die dafür sorgt, daß die Prothesenteile ihre korrekte Ausgangsstellung bis zur Beendigung des Einsetzvorgangs beibehalten. Diese Arretierungseinrichtung kann beispielsweise von einer an den Aufnahmen jeweils vorgesehenen Klemmschraube gebildet sein.

Wenn im vorliegenden Zusammenhang allgemein von Führungsschienen gesprochen wird, so sind damit sämtliche Konfigurationen gemeint, die zwangsläufig eine Ausrichtung der Prothesenteile bewirken und es gestatten, das Gerät nach einer Richtung hin von den implantierten Prothesenteilen abzuziehen. Vorzugsweise ist wenigstens eine Führungsschiene jedes Schienenpaars von einer im wesentlichen durchgehenden Nut oder Leiste gebildet. Bei dem anderen Teil genügt es dann, die Schiene durch eine Mehrzahl von Vorsprüngen anzudeuten. Wenn beispielsweise die Schiene am Rand des Prothesenteils als durchgehende Nut ausgeführt ist, kann man sich an der Aufnahme mit zwei in Abstand voneinander in die Nut eingreifenden Vorsprüngen begnügen. Oder wenn die Schiene an der Aufnahme von einer durchgehenden Leiste gebildet wird, kann man sich am Rand des Prothesenteils mit zwei Stiftpaaren oder zwei in Abstand voneinander auf einer der Leiste angeordneten Stiften und einer etwa mittig dazu auf der anderen Seite der Leiste angeordneten Stift begnügen. Die jeweils andere Schiene braucht auch nicht durchgehend ausgeführt zu sein; sie kann vielmehr an denjenigen Stellen unterbrochen sein, an denen ein Zusammenwirken mit entsprechenden Elementen des anderen Teils nicht erforderlich ist. Die Andeutung einer der beiden Schienen durch vereinzelte Vorsprünge hat auch den Vorteil, daß die erwünschte Ausrichtung der Prothesenteile gegenüber dem Einsetzgerät lediglich dann vorhanden ist, wenn die Prothesenteile korrekt in der Einsetzposition im Gerät fixiert sind. Sie endet sofort, nachdem man begonnen hat, das Gerät von den eingesetzten Implantaten zu entfernen, wodurch die Gefahr verringert wird, daß durch ungeschickte Bewegung beim Entfernen des Geräts die Implantate aus der ihnen zugedachten Position verrückt werden.

In erster Linie ist die erfindungsgemäße Anordnung für solche Prothesen vorgesehen, die mittels Knochenzement verankert werden. Bis zur Erhärtung des Zements werden die Prothesenteile durch das Einsetzgerät der korrekt aufeinander ausgerichteten Stellung gehalten.

Die Erfindung wird im folgenden näher unter Bezugnahme auf das in der Zeichnung dargestellte Ausführungsbeispiel erläutert. Darin zeigen:
- Fig. 1: eine Lateralansicht des Geräts mit darin fixierten Prothesenteilen,
- Fig. 2: einen Frontalschnitt und
- Fig. 3: eine Draufsicht.

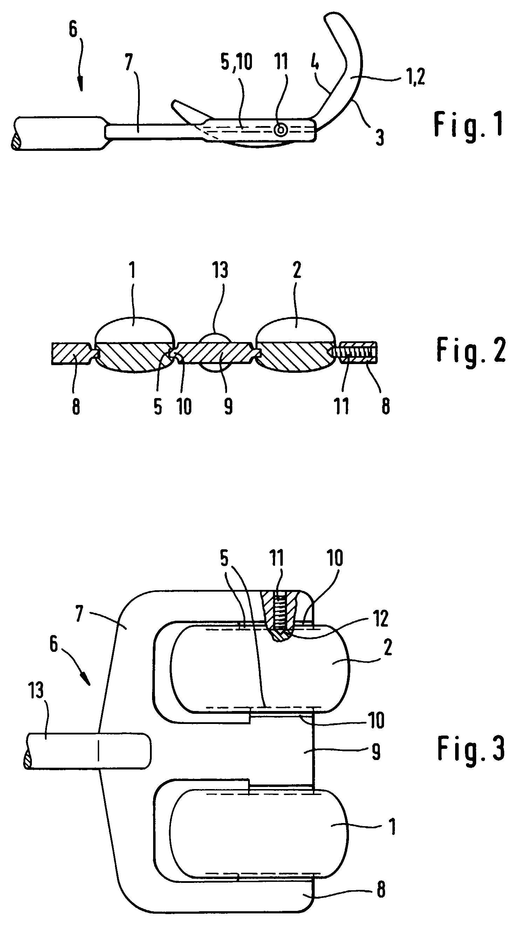

Die einzusetzende Knieprothese besteht aus den Prothesenteilen 1, 2, deren Seitenansicht aus Fig. 1 erkennbar ist und die eine Gleitfläche 3 und eine mit dem Knochen zu verbindende Fläche 4 aufweisen. Sie können zusätzlich nicht dargestellte Zapfen oder andere Oberflächenstrukturen aufweisen, die die Verankerung im Knochen und die Haftung am Zement verbessern. Das Beispiel setzt voraus, daß sie in der Seitenansicht übereinstimmen und genau parallel zueinander einzusetzen sind. Dies muß nicht so sein.

Die Prothesenteile 1, 2 sind an beiden Rändern mit Nuten 5 versehen, die parallel zueinander in AP-Richtung verlaufen. Das Einsetzinstrument 6 weist eine Doppelgabel 7 auf mit zwei Außenfingern 8 und einem Mittelfinger 9, in deren Zwischenräume die Prothesenteile 1, 2 passen. Im vorderen Abschnitt der Finger 8, 9 sind an deren einander zugewendeten Seitenflächen vorspringende Leisten 10 vorgesehen, deren Abmessungen auf die Nuten 5 der Prothesenteile abgestimmt sind und die ebenfalls parallel zueinander verlaufen. Die Prothesenteile 1, 2 können daher jeweils zwischen ein Fingerpaar 8, 9 mit ihren Nuten 5 auf die Leisten 10 aufgeschoben werden. Diese bilden die zuvor genannten, zusammenwirkenden Führungsschienen.

Die beiden Außenfinger 8 enthalten jeweils eine Madenschraube 11, deren Spitze, wie oben in Fig. 3 gezeigt, in eine entsprechende Konusbohrung 12 des zugehörigen Prothesenteils eingreift und dieses dadurch in der korrekten Einsetzlage am Einsetzgerät 6 sichert.

Das Einsetzgerät 6 weist einen Stiel 13 auf, an dem ein nicht gezeigter Griff vorgesehen ist. Die Längsrichtung des Stiels 13 und des Einsetzgeräts insgesamt stimmt mit der Richtung der Leisten 10 im wesentlichen überein.

Das Gerät gestattet es, die beiden Prothesenteile gemeinsam und in genauer gegenseitiger Ausrichtung einzusetzen und sie in der vorgesehenen Position zu halten, bis der sie befestigende Knochenzement erhärtet ist. Danach werden die Madenschrauben 11 gelöst und das Gerät kann in einer nach anterior gerichteten Bewegung weggenommen werden. Man sieht aus der Zeichnung, daß es sehr flach ausgeführt sein kann und daher im Operationsfeld nur geringen Platz einnimmt.

## Patentansprüche

1. Anordnung zum Einsetzen einer femoralen Knie-Gleitflächen-Endoprothese bestehend aus einem Einsetzgerät (6) sowie zusammenwirkenden Halteeinrichtungen an diesem und der Prothese, dadurch gekennzeichnet, daß das Einsetzgerät (6) zwei Aufnahmen (8,9) für unverbundene Condylenprothesenteile (1,2) bildet und jede Aufnahme (8.9) und jeder Condylenprothesenteil über mindestens ein Paar von Führungsschienen zusammenwirken, von denen die eine am Seitenrand des Condylenprothesenteils angeordnet ist.

2. Anordnung nach Anspruch 1, dadurch gekennzeichnet, daß die Führungsschienen (5,10) etwa in AP-Richtung verlaufen.

3. Anordnung nach Anspruch 1 oder 2, dadurch gekennzeichnet, daß die Schienenpaare (5,10) beidseitig an den Aufnahmen und den Prothesenteilen vorgesehen sind.

4. Anordnung nach einem der Ansprüche 1 bis 3, dadurch gekennzeichnet, daß eine Arretierungseinrichtung (11) zum Arretieren der Prothesenteile (1,2) in den Aufnahmen (8,9) vorgesehen ist.
